# EUROPEAN PATENT APPLICATION

(11) **EP 3 925 539 A1**
(43) Date of publication of application: **22.12.2021**
(21) Application number: 20181025.6
(22) Date of filing: 19.06.2020
(51) Int. Cl.: A61B 6/00

(54) **X-RAY IMAGING SYSTEM**

(71) Applicant: Koninklijke Philips N.V., 5656 AG Eindhoven (NL)
(72) Inventor: GRASS, Michael, 5656 AE Eindhoven (NL); KOEHLER, Thomas, 5656 AE Eindhoven (NL)
(74) Representative: Philips Intellectual Property & Standards

(57) **Abstract**

The present invention relates to an X-ray imaging system (10), comprising an X-ray image acquisition unit (20); and a processing unit (30). The X-ray image acquisition unit is configured to operate in at least one scout scan mode of operation. The X-ray image acquisition unit is configured to operate in a plurality of diagnostic image acquisition modes of operation. The X-ray image acquisition unit is configured to operate in a specific scout scan mode of operation of the at least one scout scan mode of operation to acquire a scanogram of a body part of a patient. The X-ray image acquisition unit is configured to provide the scanogram to the processing unit. The processing unit is configured to execute a trained machine learning algorithm to analyse the scanogram to select a specific diagnostic image acquisition mode of operation of the plurality of diagnostic image acquisition modes of operation, wherein the selection comprises a determination of one or more probabilities for one or more diseases or conditions associated with the body part of the patient. The X-ray image acquisition unit is configured to operate in the specific diagnostic image acquisition mode of operation to acquire diagnostic image data of the body part of the patient.

## Description

### FIELD OF THE INVENTION

The present invention relates to an X-ray imaging system, an X-ray imaging method, as well as to a computer program element and a computer readable medium.

### BACKGROUND OF THE INVENTION

Different X-ray imaging modalities find complementary and indeed different utilities for medical diagnostic purposes. X-ray Computed Tomography (CT) can frequently provide X-ray imagery required for a diagnosis. However, that diagnosis can be improved if for example spectral X-ray CT data is acquired, enabling different materials of the body to be differentiated one from the other, and the new technology of dark-field imaging has demonstrated a high potential for an improved diagnosis of pulmonary disorders especially when operating in a CT mode, and can provide important diagnostic information in particular in chest imaging for the assessment of lung diseases.

However, the acquisition of spectral data requires a reconfiguration of the source to operate in a switchable mode between different energies or for the detector to operate in a mode of operation enabling X-rays to be differentiated one from the other. Also, the acquisition of X-ray dark-field data requires a grating interferometer arrangement to be positioned in the X-ray beam line that needs to be accurately positioned.

Analysis of X-ray data acquired in one mode of operation, for example from attenuation CT data indicating that acquisition of X-ray data in a different mode of operation with a different X-ray acquisition unit should be undertaken to better confirm a diagnosis, such as transferring the patient to a dark-field CT image acquisition unit, leads to high X-ray dosage levels for the patient because they are imaged twice, and takes up a lot of time and resource.

There is a need to address these issues.

### SUMMARY OF THE INVENTION

It would be advantageous to improve X-ray imaging.

The object of the present invention is solved with the subject matter of the independent claims, wherein further embodiments are incorporated in the dependent claims. It should be noted that the following described aspects and examples of the invention apply also to the X-ray imaging system, the X-ray imaging method, as well as for the computer program element and computer readable medium.

According to a first aspect, there is provided an X-ray imaging system, comprising:
- an X-ray image acquisition unit; and
- a processing unit.

The X-ray image acquisition unit is configured to operate in at least one scout scan mode of operation. The X-ray image acquisition unit is configured to operate in a plurality of diagnostic image acquisition modes of operation. The X-ray image acquisition unit is configured to operate in a specific scout scan mode of operation of the at least one scout scan mode of operation to acquire a scanogram of a body part of a patient. The X-ray image acquisition unit is configured to provide the scanogram to the processing unit. The processing unit is configured to execute a trained machine learning algorithm to analyse the scanogram to select a specific diagnostic image acquisition mode of operation of the plurality of diagnostic image acquisition modes of operation. The selection comprises a determination of one or more probabilities for one or more diseases or conditions associated with the body part of the patient. The X-ray image acquisition unit is configured to operate in the specific diagnostic image acquisition mode of operation to acquire diagnostic image data of the body part of the patient.

In other words, a fast scout scan with minimal radiation exposure to the patient is used to determine the optimal mode of operation of an X-ray image acquisition unit that can operate in a number of different modes, where this is based on a determination of the best imaging mode matched to specific diseases or conditions of the patient has a probability of having.

Thus, for example a normal X-ray scout scan can be acquired and analysed, and it can be determined that there is a high enough probability that the patient has a disease or condition for which an X-ray dark-field mode of operation would provide the optimum image data for diagnosis purposes. Then, the X-ray image acquisition unit can be reconfigured by putting the gratings of an interferometric arrangement into the beamline in order to acquire the dark-field image data. However, it could be determined that there is no probability detected that such dark-field examination is required, and the gratings can be left out of the beamline. However, it could be determined that a disease or condition is detected for which a spectral mode of operation could be utilised, and then the X-ray source operate to provide different beam energies in a switching mode for example.

However, the system could also start with the gratings in the beamline, and a dark-field scout scan be acquired and analysed to determine if the gratings should be left in the beamline and the diagnostic dark-field scan obtained, or the gratings be removed and attenuation or spectral CT scan undertaken.

The reconfiguring of the X-ray acquisition unit can be carried out by a medical operator. Thus, the processing unit can relay a message to a medical operator, who then reconfigures the X-ray acquisition unit. However, the reconfiguring can be automatic, with the processing unit controlling the reconfiguration directly upon selection of the specific diagnostic image acquisition mode of operation.

In an example, the at least one scout scan mode of operation comprises an X-ray attenuation scout scan mode of operation.

In an example, the at least one scout scan mode of operation comprises an X-ray dark-field scout scan mode of operation.

In an example, the plurality of diagnostic image acquisition modes of operation comprises an X-ray attenuation CT mode of operation, and X-ray spectral attenuation CT mode of operation.

In an example, the plurality of diagnostic image acquisition modes of operation comprises an X-ray dark-field CT mode of operation.

In an example, the X-ray image acquisition unit comprises an interferometer arrangement configured to be moved into an X-ray beam line of the X-ray image acquisition unit and configured to be moved out of the X-ray beam line of the X-ray image acquisition unit.

In an example, the processing unit is configured to control movement of the interferometer arrangement into the X-ray beam line of the X-ray image acquisition unit on the basis of the selected specific diagnostic image acquisition mode of operation.

In an example, the processing unit is configured to control movement of the interferometer arrangement out of the X-ray beam line of the X-ray image acquisition unit on the basis of the selected specific diagnostic image acquisition mode of operation.

In this manner, if the x-ray acquisition unit operates in a scout scan mode with the gratings out of the beamline and analysis of the scanogram indicates a high enough probability of disease or condition for which a dark-field scan would be appropriate, the gratings can be automatically positioned into the beam line and dark-field image data acquired. Alternatively, the X-ray acquisition unit could operate in a dark-field scout scan mode of operation and analysis of the dark-field scanogram indicate that a dark-field diagnostic image scan is not required, and the gratings can be automatically removed from the beam line and an attenuation or spectral CT scan can then be acquired.

In an example, the processing unit is configured to control movement of the interferometer arrangement into the X-ray beam line of the X-ray image acquisition unit on the basis of the specific scout scan mode of operation.

In an example, the processing unit is configured to control movement of the interferometer arrangement out of the X-ray beam line of the X-ray image acquisition unit on the basis of the specific scout scan mode of operation.

In this manner, an operator can indicate how the system is to operate in the scout scan mode, whether an attenuation scanogram or a dark-field scanogram is to be acquired, and the system automatically configures itself in preparedness for such an initial scan.

In an example, the plurality of diagnostic image acquisition modes of operation comprises an X-ray attenuation CT mode of operation. The processing unit is configured to select a reconstruction protocol for the diagnostic image data of the body part, and the selection comprises utilization of the determined one or more probabilities for the one or more diseases or conditions associated with the body part of the patient.

In an example, the plurality of diagnostic image acquisition modes of operation comprises an X-ray spectral attenuation CT mode of operation. The processing unit is configured to select a reconstruction protocol for the diagnostic image data of the body part, and the selection comprises utilization of the determined one or more probabilities for the one or more diseases or conditions associated with the body part of the patient.

In other words, not only can the diagnostic image scan mode be selected based on analysis of scout scan scanogram with respect to the determined probability of the patient suffering from a disease or condition, but that determined probability can be used in selecting a reconstruction protocol for CT data. In this manner, a completely automated self-contained operative system is provided.

In an example, the machine learning algorithm has been trained on a plurality of reference images and ground truth information relating to one or more reference patients having one or more diseases or conditions associated with a body part of the patient, such as a lung disease, for instance one or more of: Chronic Obstructive Pulmonary diseases (COPD), fibrosis, emphysema, pneumothorax, inflammation of the lung, lung cancer, and Covid 19.

According to a second aspect, there is provided an X-ray imaging method, comprising:
- operating an X-ray image acquisition unit in a specific scout scan mode of operation of at least one scout scan mode of operation to acquire a scanogram of a body part of a patient;
- providing the scanogram to a processing unit;
- executing by the processing unit a trained machine learning algorithm to analyse the scanogram to select a specific diagnostic image acquisition mode of operation of a plurality of diagnostic image acquisition modes of operation of the X-ray image acquisition unit, wherein the selection comprises a determination of one or more probabilities for one or more diseases or conditions associated with the body part of the patient; and
- operating the X-ray image acquisition unit in the specific diagnostic image acquisition mode of operation to acquire diagnostic image data of the body part of the patient.

According to another aspect, there is provided a computer program element controlling a system as previously described which, if the computer program element is executed by a processing unit, is adapted to perform the method steps as previously described.

According to another aspect, there is provided a computer readable medium having stored computer element as previously described.

The computer program element, can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an X-ray imaging system;
Fig. 2 shows a schematic set up of an example of an X-ray imaging method;
Fig. 3 shows a diagrammatical representation of a detailed exemplar workflow of an X-ray imaging method; and
Fig. 4 shows a schematic set up of an example of a dark-field imaging system.

### DETAILED DESCRIPTION OF EMBODIMENTS

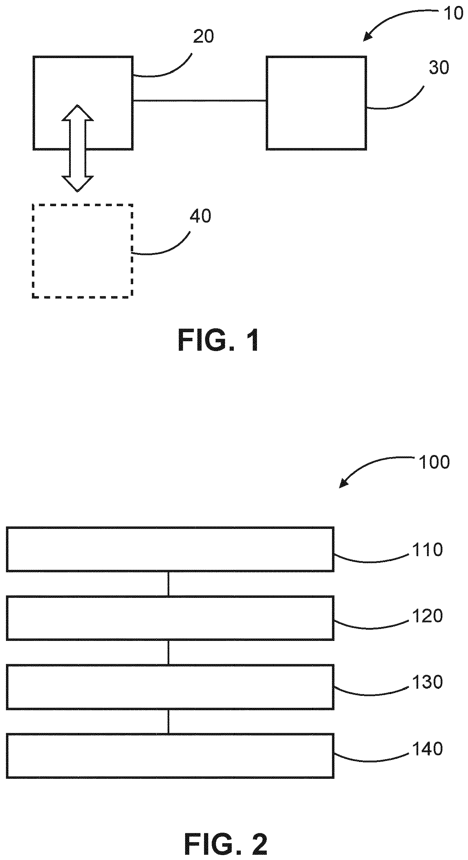

Fig. 1 shows a schematic example of an X-ray imaging system 10, where essential features are represented by solid lines and optional features by hashed lines. The system 10 comprises an X-ray image acquisition unit 20, and a processing unit 30. The X-ray image acquisition unit is configured to operate in at least one scout scan mode of operation. The X-ray image acquisition unit is configured to operate in a plurality of diagnostic image acquisition modes of operation. The X-ray image acquisition unit is configured to operate in a specific scout scan mode of operation of the at least one scout scan mode of operation to acquire a scanogram of a body part of a patient. The X-ray image acquisition unit is configured to provide the scanogram to the processing unit. The processing unit is configured to execute a trained machine learning algorithm to analyse the scanogram to select a specific diagnostic image acquisition mode of operation of the plurality of diagnostic image acquisition modes of operation. The selection of the specific diagnostic image acquisition mode of operation by the trained machine learning algorithm comprises a determination of one or more probabilities for one or more diseases or conditions associated with the body part of the patient. The X-ray image acquisition unit is configured to operate in the specific diagnostic image acquisition mode of operation to acquire diagnostic image data of the body part of the patient.

In an example, the processing unit is configured to control the reconfiguration of the X-ray image acquisition unit to operate in the specific diagnostic image acquisition mode of operation.

According to an example, the at least one scout scan mode of operation comprises an X-ray attenuation scout scan mode of operation.

According to an example, the at least one scout scan mode of operation comprises an X-ray dark-field scout scan mode of operation.

According to an example, the plurality of diagnostic image acquisition modes of operation comprises an X-ray attenuation CT mode of operation, and X-ray spectral attenuation CT mode of operation.

According to an example, the plurality of diagnostic image acquisition modes of operation comprises an X-ray dark-field CT mode of operation.

According to an example, the X-ray image acquisition unit comprises an interferometer arrangement 40 configured to be moved into an X-ray beam line of the X-ray image acquisition unit and configured to be moved out of the X-ray beam line of the X-ray image acquisition unit.

According to an example, the processing unit is configured to control movement of the interferometer arrangement into the X-ray beam line of the X-ray image acquisition unit on the basis of the selected specific diagnostic image acquisition mode of operation.

According to an example, the processing unit is configured to control movement of the interferometer arrangement out of the X-ray beam line of the X-ray image acquisition unit on the basis of the selected specific diagnostic image acquisition mode of operation.

According to an example, the processing unit is configured to control movement of the interferometer arrangement into the X-ray beam line of the X-ray image acquisition unit on the basis of the specific scout scan mode of operation.

According to an example, the processing unit is configured to control movement of the interferometer arrangement out of the X-ray beam line of the X-ray image acquisition unit on the basis of the specific scout scan mode of operation.

In an example, the processing unit is configured to control an x-ray source of the X-ray acquisition unit to operate in a multi-energy switching mode of operation on the basis of the selected specific diagnostic image acquisition mode of operation.

In an example, the processing unit is configured to control a detector of the X-ray acquisition unit to operate in a multi-energy resolving mode of operation on the basis of the selected specific diagnostic image acquisition mode of operation.

In an example, the processing unit is configured to determine that a detector of the X-ray acquisition unit needs to be replaced with a detector having a multi-energy resolving mode of operation on the basis of the selected specific diagnostic image acquisition mode of operation.

According to an example, the plurality of diagnostic image acquisition modes of operation comprises an X-ray attenuation CT mode of operation, and the processing unit is configured to select a reconstruction protocol for the diagnostic image data of the body part. The selection can comprise utilization of the determined one or more probabilities for the one or more diseases or conditions associated with the body part of the patient.

According to an example, the plurality of diagnostic image acquisition modes of operation comprises an X-ray spectral attenuation CT mode of operation, and the processing unit is configured to select a reconstruction protocol for the diagnostic image data of the body part. The selection can comprise utilization of the determined one or more probabilities for the one or more diseases or conditions associated with the body part of the patient.

According to an example, the machine learning algorithm has been trained on a plurality of reference images and ground truth information relating to one or more reference patients having one or more diseases or conditions associated with a body part of the patient, such as a lung disease, for instance one or more of: Chronic Obstructive Pulmonary diseases (COPD), fibrosis, emphysema, pneumothorax, inflammation of the lung, lung cancer, and Covid 19.

Fig. 2 shows an X-ray imaging method 100 in its basic steps. The method 100 comprises:
- operating 110 an X-ray image acquisition unit in a specific scout scan mode of operation of at least one scout scan mode of operation to acquire a scanogram of a body part of a patient;
- providing 120 the scanogram to a processing unit;
- executing 130 by the processing unit a trained machine learning algorithm to analyse the scanogram to select a specific diagnostic image acquisition mode of operation of a plurality of diagnostic image acquisition modes of operation of the X-ray image acquisition unit, wherein the selection comprises a determination of one or more probabilities for one or more diseases or conditions associated with the body part of the patient; and
- operating 140 the X-ray image acquisition unit in the specific diagnostic image acquisition mode of operation to acquire diagnostic image data of the body part of the patient.

In an example, method comprises controlling by the processing unit the reconfiguration of the X-ray image acquisition unit to operate in the specific diagnostic image acquisition mode of operation.

In an example, the at least one scout scan mode of operation comprises an X-ray attenuation scout scan mode of operation.

In an example, the at least one scout scan mode of operation comprises an X-ray dark-field scout scan mode of operation.

In an example, the plurality of diagnostic image acquisition modes of operation comprises an X-ray attenuation CT mode of operation, and X-ray spectral attenuation CT mode of operation.

In an example, the plurality of diagnostic image acquisition modes of operation comprises an X-ray dark-field CT mode of operation.

In an example, the X-ray image acquisition unit comprises an interferometer arrangement.

In an example, the method comprises controlling by the processing unit movement of the interferometer arrangement into the X-ray beam line of the X-ray image acquisition unit on the basis of the selected specific diagnostic image acquisition mode of operation.

In an example, the method comprises controlling by the processing unit movement of the interferometer arrangement out of the X-ray beam line of the X-ray image acquisition unit on the basis of the selected specific diagnostic image acquisition mode of operation.

In an example, the method comprises controlling by the processing unit a movement of the interferometer arrangement into the X-ray beam line of the X-ray image acquisition unit on the basis of the specific scout scan mode of operation.

In an example, the method comprises controlling by the processing unit a movement of the interferometer arrangement out of the X-ray beam line of the X-ray image acquisition unit on the basis of the specific scout scan mode of operation.

In an example, the method comprises controlling by the processing unit an X-ray source of the X-ray acquisition unit to operate in a multi-energy switching mode of operation on the basis of the selected specific diagnostic image acquisition mode of operation.

In an example, the method comprises controlling by the processing unit a detector of the X-ray acquisition unit to operate in a multi-energy resolving mode of operation on the basis of the selected specific diagnostic image acquisition mode of operation.

In an example, the method comprises determining by the processing unit that a detector of the X-ray acquisition unit needs to be replaced with a detector having a multi-energy resolving mode of operation on the basis of the selected specific diagnostic image acquisition mode of operation.

In an example, the plurality of diagnostic image acquisition modes of operation comprises an X-ray attenuation CT mode of operation, and wherein the method comprises selecting by the processing unit a reconstruction protocol for the diagnostic image data of the body part, wherein the selection comprises utilizing the determined one or more probabilities for the one or more diseases or conditions associated with the body part of the patient.

In an example, the plurality of diagnostic image acquisition modes of operation comprises an X-ray spectral attenuation CT mode of operation, and wherein the method comprises selecting by the processing unit a reconstruction protocol for the diagnostic image data of the body part, wherein the selection comprises utilizing the determined one or more probabilities for the one or more diseases or conditions associated with the body part of the patient.

In an example, the machine learning algorithm has been trained on a plurality of reference images and ground truth information relating to one or more reference patients having one or more diseases or conditions associated with a body part of the patient, such as a lung disease, for instance one or more of: Chronic Obstructive Pulmonary diseases (COPD), fibrosis, emphysema, pneumothorax, inflammation of the lung, lung cancer, and Covid 19.

Reference above is made to a "scanogram". Here, this means a scout scan. A scout scan in general is a scan where the CT gantry does not rotate. The detector is collimated to typically two rows and the table upon which the patient is lying is moved through the system and data are continuously acquired. The data are then re-arranged to provide a result that looks similar to an X-ray radiography image. For a dark-field scout scan, the collimation is opened to illuminate a couple of detector rows and gratings are placed in the beam. Data acquisition includes again moving the patient through the arrangement without gantry rotation. During phase retrieval, the cone-angle can typically be ignored. Further detail on a dark-field scout scan can be found in C. Kottler et al: rev. Sci. Instrum. 78 043710 (2007).

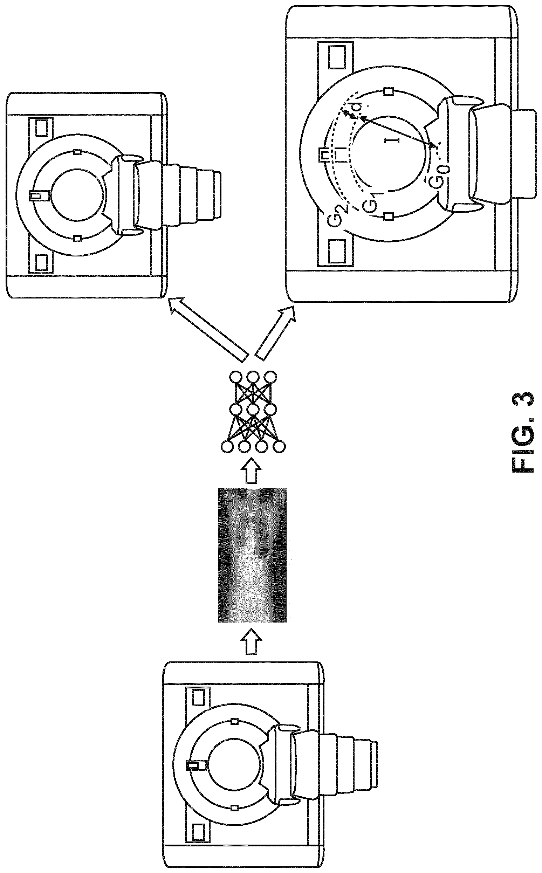

The X-ray imaging system and method are now described in specific details, where reference is made to Figs. 3-4.

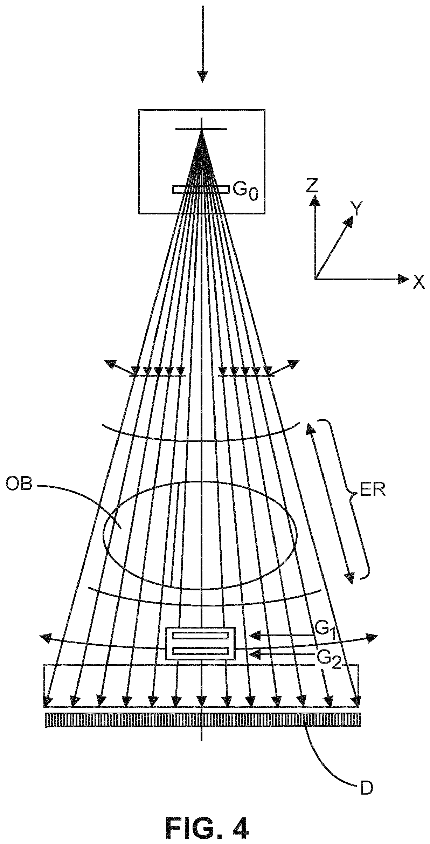

Fig. 3 shows a diagrammatical representation of a detailed exemplar workflow of an X-ray imaging method being applied to a reconfigurable X-ray system, where an X-ray acquisition unit can be configured as an attenuation CT image acquisition unit or configured as a dark-field image acquisition unit. In Fig. 3 scanogram acquisition is undertaken by an x-ray image acquisition unit operating in a scout scan mode, represented on the left. After scanogram acquisition, automatic disease likelihood detection is performed on the scanogram data, shown in the centre where a trained neural network or other trained machine learning algorithm is utilized to analyse the scout scan data, shown in the middle images. Subsequently the scanner reconfiguration is adapted corresponding to the detected disease (upper right - conventional CT imaging, lower right - dark-field CT imaging with a three grating interferometer discussed in more detail with respect to Fig.4, with G0, G1 and G2 gratings, where phase and analyzer gratings G1 and G2 are shown separated by a distance d). The reconfiguration of the scanner can be carried out by a medical operative, who is informed after the scanogram has been analysed that the scanner should be reconfigured, or the scanner can automatically reconfigure itself as required.

Thus the basis behind the new method was the realisation that modern AI based methods for disease detection could be applied to scanograms as they are acquired to determine a probability of certain diseases from the X-ray images. The scanogram analysis is valid for lung disease, but also a multitude of other diseases are detectable with high accuracy. This is achieved by separate training based on scanogram data with known diagnosis or by post-training of existing networks on a smaller scanogram data base. It is to be noted that in lung applications, dark-field imaging may sometimes be preferred to conventional CT imaging due to its excellent imaging capabilities for e.g. COPD and fibrosis detection and diagnosis. Thus, scanogram data of various body parts of patients having known diseases and conditions is used to train a machine learning algorithm such as a neural network. Then the neural network when presented with a new scanogram of a patient's body part can analyse that to provide probabilities that the scanogram data of the body part being is indicative of different disease or conditions. The different diseases and conditions can then be linked to optimal imaging modes: attenuation CT, spectral CT, dark-field CT that would be best utilized to provide image data to confirm a diagnosis. From this information, the required mode of operation of an X-ray image acquisition unit can be selected.

Therefore, the further realization was that the AI based analysis of the attenuation scanogram enabled a low dosage to be used to determine if a follow on full scan should be in an attenuation CT mode, or whether there was a detected probability of a disease or condition that warranted reconfiguration of the X-ray image acquisition unit to an attenuation spectral CT unit or to a dark-field CT imaging acquisition unit, which requires a reconfiguration of the conventional CT system by changing the mode of operation of the X-ray source/detector or moving the gratings required for interferometric imaging into the X-ray beam. Thus, the new methodology was based on the automatically triggering of dark-field CT scanning and hardware reconfiguration based on high disease probability detected from scanogram data using neural networks. As discussed above, although the selection of imaging mode is automatic, the actual reconfiguration can be carried out manually or can also be carried out automatically.

Rather than acquiring a scanogram in a conventional X-ray mode, a scanogram in the dark-field mode can be acquired. Then a decision can be made based on the neural network analysis of the scanogram and resultant probabilities of diseases or conditions being detected to undertake a full dark-field CT scan by keeping the gratings in the beam line, or remove the gratings and carry out a normal attenuation CT scan or indeed an attenuation spectral CT scan.

Thus, scanogram can also be used to trigger that the X-ray source operate in a switching mode such that spectral CT attenuation data can be acquired, or that the detector operate in an energy resolving mode for the same purposes or even that a special two-layer detector take the place of the normal detector. Depending upon the probability of certain diseases being present, different image reconstruction protocols find best utility for CT image data whether monochromatic or spectral, and the neural network analysis can also be used to select the preferred reconstruction algorithm or protocol.

Thus, a method is provided to automatically trigger dark-field CT scanning and hardware reconfiguration based on high disease probability detected from scanogram data using neural networks. This optimizes the workflow in CT based lung imaging / screening by using scanogram based disease/condition determination. This leads to reduced delays due to automatic scanner reconfiguration and to optimization of the quality of care.

### Reconfiguration to a dark-field X-ray image acquisition unit

For the acquisition of the dark-field data, a two (Talbot type) or three-grating (Talbot-Lau type) interferometer is introduced into the X-ray beam line to reconfigured the X-ray acquisition unit. The gratings are normally termed G0, G1 and G2 gratings. An exemplar system is shown in Fig. 4, where typically G0 and G2 are absorber gratings and G1 is a phase grating, and where an object OB is placed within an examination region ER. The source grating G0, can be used to make radiation from the source more coherent but is not always necessary, and gratings G1 and G2 are normally termed phase and analyzer gratings. Subsequently, for a so-called full field system, one of the two gratings G1 or G2 is moved perpendicular to the grating lamellae relative to the other gratings in a number of steps (so-called stepping), and if the source grating G0 is utilized it can be this grating that is stepped laterally (where laterally means perpendicular to the grating direction). Thereby, for each new grating position an image is recorded on the detector D. Comparison of the image sequence acquired with and without a sample in the beam, allows to calculate the three imaging signals: transmission or attenuation (conventional X-ray image), phase contrast image, and dark-field image. These gratings generate a fringe pattern on top of the conventional transmission image, and for example the dark-field signal is calculated as the loss of contrast of this fringe pattern. The fringe-pattern, which is analyzed in dark-field and phase contrast imaging, is a fine structure in the micrometer range. Using an analyzer grating with the same periodicity, a Moire-pattern can be measured with the detector. Any movement of one or more interferometer components, such as the analyzer grating, in the length scale of its period changes the phase of the Moire-pattern. Instead of using a full-field system and phase stepping, a scanning type of system can be used as described e.g. in US10485492 B2, GB3314576 or JP6415759 B2 for a dark-field CT system.

Thus, a sample or object, the body in Fig. 4, modulates attenuation, refraction, and small angle scattering information onto the radiation. To separate phase information from other contributions to the signal, such as attenuation by the sample, inhomogeneous illumination or imperfections of the gratings, a phase "stepping" approach is utilized. One of the gratings (either G1 or G2 - or G0 if present) is scanned along the transverse direction over at least one period of the grating, and for every point of the scan an image is taken (it is to be noted that the intensity modulation mask is applied to this scan). The resultant phase contrast, dark field, and attenuation data then oscillates sinusoidal, with and without the sample, and this can be utilized to determine the dark field, phase contrast and attenuation images using a phase retrieval algorithm. However, now the source and/or gratings are simultaneously rotated to change the line of sight through the sample or object, not shown in Fig. 4, to enable dark-field CT data to be acquired. Further detail on the standard phase stepping approach can be found in the paper by Weitkamp et al, Optics Express, Vol. 13, No. 16, (2005) 6296-6304.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging system (10), comprising:
- an X-ray image acquisition unit (20); and
- a processing unit (30);
wherein, the X-ray image acquisition unit is configured to operate in at least one scout scan mode of operation;
wherein, the X-ray image acquisition unit is configured to operate in a plurality of diagnostic image acquisition modes of operation;
wherein, the X-ray image acquisition unit is configured to operate in a specific scout scan mode of operation of the at least one scout scan mode of operation to acquire a scanogram of a body part of a patient;
wherein, the X-ray image acquisition unit is configured to provide the scanogram to the processing unit;
wherein, the processing unit is configured to execute a trained machine learning algorithm to analyse the scanogram to select a specific diagnostic image acquisition mode of operation of the plurality of diagnostic image acquisition modes of operation, wherein the selection comprises a determination of one or more probabilities for one or more diseases or conditions associated with the body part of the patient; and
wherein, the X-ray image acquisition unit is configured to operate in the specific diagnostic image acquisition mode of operation to acquire diagnostic image data of the body part of the patient.

2. System according to claim 1, wherein the at least one scout scan mode of operation comprises an X-ray attenuation scout scan mode of operation.

3. System according to any of claims 1-2, wherein the at least one scout scan mode of operation comprises an X-ray dark-field scout scan mode of operation.

4. System according to any of claims 1-3, wherein the plurality of diagnostic image acquisition modes of operation comprises an X-ray attenuation CT mode of operation, and X-ray spectral attenuation CT mode of operation.

5. System according to any of claims 1-4, wherein the plurality of diagnostic image acquisition modes of operation comprises an X-ray dark-field CT mode of operation.

6. System according to any of claims 1-5, wherein the X-ray image acquisition unit comprises an interferometer arrangement (40) configured to be moved into an X-ray beam line of the X-ray image acquisition unit and configured to be moved out of the X-ray beam line of the X-ray image acquisition unit.

7. System according to claim 6, wherein the processing unit is configured to control movement of the interferometer arrangement into the X-ray beam line of the X-ray image acquisition unit on the basis of the selected specific diagnostic image acquisition mode of operation.

8. System according to any of claims 6-7, wherein the processing unit is configured to control movement of the interferometer arrangement out of the X-ray beam line of the X-ray image acquisition unit on the basis of the selected specific diagnostic image acquisition mode of operation.

9. System according to any of claims 6-8, wherein the processing unit is configured to control movement of the interferometer arrangement into the X-ray beam line of the X-ray image acquisition unit on the basis of the specific scout scan mode of operation.

10. System according to any of claims 6-9, wherein the processing unit is configured to control movement of the interferometer arrangement out of the X-ray beam line of the X-ray image acquisition unit on the basis of the specific scout scan mode of operation.

11. System according to any of claims 1-10, wherein the plurality of diagnostic image acquisition modes of operation comprises an X-ray attenuation CT mode of operation, and wherein the processing unit is configured to select a reconstruction protocol for the diagnostic image data of the body part, wherein the selection comprises utilization of the determined one or more probabilities for the one or more diseases or conditions associated with the body part of the patient.

12. System according to any of claims 1-11, wherein the plurality of diagnostic image acquisition modes of operation comprises an X-ray spectral attenuation CT mode of operation, and wherein the processing unit is configured to select a reconstruction protocol for the diagnostic image data of the body part, wherein the selection comprises utilization of the determined one or more probabilities for the one or more diseases or conditions associated with the body part of the patient.

13. System according to any of claims 1-12, wherein the machine learning algorithm has been trained on a plurality of reference images and ground truth information relating to one or more reference patients having one or more diseases or conditions associated with a body part of the patient, such as a lung disease, for instance one or more of: Chronic Obstructive Pulmonary diseases (COPD), fibrosis, emphysema, pneumothorax, inflammation of the lung, lung cancer, and Covid 19.

14. An X-ray imaging method (100), comprising:
- operating (110) an X-ray image acquisition unit in a specific scout scan mode of operation of at least one scout scan mode of operation to acquire a scanogram of a body part of a patient;
- providing (120) the scanogram to a processing unit;
- executing (130) by the processing unit a trained machine learning algorithm to analyse the scanogram to select a specific diagnostic image acquisition mode of operation of a plurality of diagnostic image acquisition modes of operation of the X-ray image acquisition unit, wherein the selection comprises a determination of one or more probabilities for one or more diseases or conditions associated with the body part of the patient; and
- operating (140) the X-ray image acquisition unit in the specific diagnostic image acquisition mode of operation to acquire diagnostic image data of the body part of the patient.

15. A computer program element for controlling a system of any of claims 1-13, which when executed by a processor is configured to carry out the method of claim 14.
